Europäisches Patentamt

European Patent Office

Office européen des brevets

(19)

(11) Numéro de publication: **0 082 072 B1**

(12) **FASCICULE DE BREVET EUROPEEN**

(45) Date de publication du fascicule du brevet:
21.08.85

(51) Int. Cl.⁴: **A 61 K 39/112**

(21) Numéro de dépôt: 82402249.5

(22) Date de dépôt: 09.12.82

(54) Procédé de production d'un vaccin biochimique contre les fièvres à salmonelles.

(30) Priorité: 10.12.81 FR 8123067

(43) Date de publication de la demande:
22.06.83 Bulletin 83/25

(45) Mention de la délivrance du brevet:
21.08.85 Bulletin 85/34

(84) Etats contractants désignés:
AT BE CH DE FR GB IT LI LU NL SE

(56) Documents cités:
BIOLOGICAL ABSTRACTS, vol. 63, 15 mars 1977, no. 33776, page 3317; E.J. JOHNSON et al.: "Anatomical locus of the common enterobacterial antigen"
BIOLOGICAL ABSTRACTS, vol. 66, no. 4, page 2186, no. 22334; J.E. SIPPEL et al.: "Outer membrane protein antigens in an enzyme-linked immunosorbent assay for Salmonella enteric fever and memingococcal meningtis"
CHEMICAL ABSTRACTS, vol. 91, no. 25, 17 decembre 1979, page 501, no. 209139a, Columbus Ohio (USA); S.B. SVENSON et al.: "Artificial Salmonella vaccines: O-antigenic oligosaccharide-protein conjugates induce protection against infection with Salmonella typhimurium"
BIOLOGICAL ABSTRACTS, vol. 69, no. 2, 1980, page 1023, no. 9624; N. KUUSI et al.: "Immunization with
major outer membrane proteins in experimental salmonellosis of mice"
BIOLOGICAL ABSTRACTS, vol. 73, no. 6, 1982, no. 39201; N. KUUSI et al.: "Immunization with major outer membrane protein (porin) preparations in experimental murine salmonellosis: Effect of lipopolysaccharide"
CHEMICAL ABSTRACTS, vol. 96, no. 5, 1er février 1982, page 313, no. 31280c, Columbus Ohio (USA); M.A. TUFANO et al.: "Receptorial and antigenic properties of precursors proteins to 34K and 36K porins of Salmonella typhimurium"

(73) Titulaire: L'AIR LIQUIDE, SOCIETE ANONYME POUR L'ETUDE ET L'EXPLOITATION DES PROCEDES GEORGES CLAUDE, 75, Quai d'Orsay, F-75321 Paris Cedex 07 (FR)

(72) Inventeur: Girardon, Philippe, 19, rue du Hazard, F-78000 Versailles (FR)
Inventeur: Amen, Jean, 108, bd de la Reine, F-78000 Versailles (FR)

(74) Mandataire: Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)

**0 082 072**

## Description

La présente invention concerne l'obtention d'un vaccin biochimique contre les fièvres à Salmonelles, fièvres typhoïdiques et paratyphoïdiques.

La menace d'épidémie de fièvres à Salmonelles est permanente dans le monde comme en témoigne les relevés épidémiologiques; et la gravité de l'extension des épidémies dues aux diverses Salmonelles ont toutes pour origine, une résistance de plus en plus grande des germes aux antibiotiques.

On sait depouis longtemps qu'il n'existe aucun parallélisme entre le taux des anticorps agglutinants anti-O, anti-H ou anti Vi, mis en évidence dans le cas des Salmonelloses et le degré de l'immunité acquise vis à vis de ces bactéries. Actuellement, le seul critère valable d'efficacité vaccinale demeure la protection de l'individu ou de l'animal vis à vis de la bactérie infectieuse homologue.

Plusieurs types de vaccins ont déjà été proposés, cependant ces vaccins ne sont pas sans présenter divers inconvénients et risques. Ainsi les vaccins inactivés injectables (TAB) ne sont pas sans danger pour les sujets qui les reçoivent, et ceci pour une efficacité relative par rapport à une dose d'épreuve ne dépassant pas $10^5$ bactéries pathogènes. Par contre, les vaccins buccaux, et plus particulièrement ceux qui contiennent des bactéries vivantes, se révèlent nettement plus efficaces mais leur utilisation pose encore un certain nombre de problèmes d'ordre pratique inhérents à des difficultés de conservation, et d'ordre psychologique.

On a considéré que l'utilisation de fractions purifiées protectrices extraites de bactéries résoudrait de manière satisfaisante le problème de la vaccination contre les Salmonelles. Et en sachant que toutes les bactéries peuvent être regardées comme constituées par des »mosaïques d'antigènes«, la membrane externe de la paroi ectoplasmique apparaissant, chez les Salmonelles (gram négatives) comme le support anatomique de l'antigène vaccinant, il a été recherché un moyen d'extraction préférentielle de cet antigène vaccinant.

Divers travaux, notamment ceux de O. Westphal et al., du Max Planck Institut, publiés notamment dans Zentralbl. Bacteriol; Mikrobiol. Hyg. 1 ABt Orig. a. med. mikrobiol infektionskr. Parasitol 248 (1), 1980, 64—80, ont montré l'utilisation de guanidine thiocyanate, urée et de tampon véronal dans l'extraction de molécules à base de protéines, en particulier à partir de bactéries gram négatives telles Salmonella typhimurium et de S. minnesota et des formes mutantes 5 R.

Dans le cadre d'une technique par fermentation, extraction et purification, il a été mis au point un procédé permettant d'obtenir un vaccin biochimique actif contre les fièvres à Salmonelles, affectant les êtres humains et animaux, aussi efficace que le vaccin vivant, de conservation aisée et de très longue durée et d'administration facile en injection ou par voie buccale.

Le procédé de l'invention est caractérisé par une séquence des diverses phases préparatives suivantes.

Dans un premier stade on met en oeuvre la fermentation en milieu liquide convenablement adapté d'une souche avirulente, telle Salmonella typhimurium M 206. Il est avantageux de maintenir la température du milieu de fermentation dans des conditions strictes de température comprise entre 33 et 35° C, la température de 34° C étant particulièrement adaptée au développement de Salmonella typhimurium. La fermentation est conduite en moyenne aérobiose pendant une durée de l'ordre de 24 heures; le pH du milieu étant ajusté entre 7 et 7,7.

On procède ensuite à une centrifugation à faible vitesse, ayant pour objet la séparation du liquide et des bactéries sous forme de culot bactérien, qui est lavé.

Puis on extrait les antigènes membranaires par un solvant sélectif déterminé et dans des conditions de pH et de durée bien précises.

On réalise l'extraction des antigènes membranaires vaccinants par mise en contact du culot bactérien avec un solvant de la classe des tris (hydroxyalcoyl) aminoalcanes inférieurs. Le tris (hydroxyméthyl) aminométhane est particulièrement adapté à l'extraction des antigènes membranaires dans le cadre du procédé.

Le solvant est utilisé à concentration de 0,05 M et le milieu d'extraction est tamponné, le pH étant régulé entre 8,4 et 8,6 par ajustage avec un acide fort. L'extraction est effectuée sur le culot bactérien sous agitation permanente, à une température réfrigérée, pour éviter tout développement enzymatique ou microbien, de préférence entre O et +4° C. La durée d'extraction des antigènes sous-agitation du culot bactérien au contact du milieu tampon-solvant est au moins de soixante heures, de préférence de l'ordre de 70 heures.

On effectue ensuite une décantation par centrifugation, et une purification et fractionnement par ultrafiltration du surnageant sur membranes à différents seuils de coupure allant de 10 000 à 300 000 pour obtenir les différentes fractions de poids moléculaires recherchés. On peut procéder à une dialyse de la fraction de plus faible poids moléculaire afin d'éliminer les sels.

Dans une dernière phase on lyophilise, selon les techniques connues la fraction antigénique vaccinante.

Selon une variante avantageuse on peut conduire l'extraction des antigènes par un solvant en présence d'un tensio-actif, tels les tensio-actifs de la série des polyoxyéthylènes et des polyoxyéthanols, utilisé à une concentration de l'ordre de 1% par rapport au milieu extractif. A titre de tensio-actif convenant à l'amélioration de l'extraction des antigènes vaccinants on peut citer les polyoxyéthylène-

2

0 082 072

sorbates, vendus sous la marque commerciale »Tween«, les polyoxyéthylènelaurylesters, commercialisés sous la marque »Bridj« et le polyéthoxyéthanol connu sous la marque commerciale »Triton«. L'emploi d'un tensio-actif est bénéfique du point de vue de l'activité; toutefois, il convient de noter que l'emploi d'un tensio-actif au cours de l'extraction peut conduire à certaines difficultés de purification ultérieure, si la production a pour but un antigène purifié. Dans ce cas, l'extrait des antigènes membranaires vaccinants après purification et fractionnement par ultrafiltration est soumis à une purification poussée par isoélectrofocalisation ou au plan industriel sur résine échangeuses d'ions.

Les vaccins biochimiques lyophilisés obtenus selon l'invention, dont le poids moléculaire de la fraction antigénique vacinante est supérieur à 50 000 sont utilisables dans la vaccination des êtres humains et des animaux, et vaccinent les souris aussi efficacement que le vaccin vivant. Les vaccins dont le poids moléculaire de la fraction antigénique est supérieur à 300 000 sont d'utilisation très intéressante, vis à vis du traitement des fièvres à Salmonelles, typhoïdiques et paratyphoïdiques, car on peut envisager ce type de vaccin à Salmonella typhi. Ces vaccins peuvent être prescrits par voie injectable ou buccale. Du fait de leur production sous forme lyophilisée leur conservation est aisée et de très longue durée.

Il est donné ci-après des exemples qui illustrent l'invention à titre non limitatif.


Exemple 1

On conduit une fermentation de Salmonella typhimurium M 206 en fermenteur de 15 litres de volume utile, dans un milieu type bouillon Eugon, de composition pour 1 litre:

| | |
|---|---|
| Tryptone | 15 g |
| Peptone papaïnique de soja | 5 g |
| Glucose | 5 g |
| NaCl | 4 g |
| L. cystine | 0,3 g |
| Sulfite de sodium | 0,2 g |
| Citrate de sodium | 1 g |

La culture est conduite à une température de 34°C, avec une aération 0,5 VVM, le pH est ajusté à 7,7, au départ on observe peu d'acidification. La durée de fermentation est de 24 heures. On procède à une centrifugation à faible vitesse, le culot est lavé à l'eau, déminéralisé puis pesé.

Sur 30 g de culot bactérien (poids humide), on procède à l'extraction par le solvant suivant tampon tris (hydroxyméthyl) aminométhane (0,05 M), HCl à pH 8,5 (200 ml), à une température de +4°C, sous agitation permanente, et à pH régulé à 8,5. Le rendement de l'extraction contrôlé de l'absorption à 206 nanomètres est maximum au bout d'un temps moyen voisin de 67 à 72 heures. Au-delà de cette durée, on observe un palier sans amélioration du rendement d'extraction.

On soumet à une décantation par centrifugation très légère, puis à une ultrafiltration sur membranes de seuils de coupure de 50 000 et 10 000 Daltons. On obtient les fractions suivantes:

| fractions antigéniques | Poids |
|---|---|
| Pm > 50 000 | 0,596 g |
| 10 000 < PM < 50 000 | 0,039 g |
| PM < 10 000 | 0,77 g |

Avec chacune des trois fractions séparées on procède à la vaccination de trois lots de 100 souris par administration par voie buccale de 200 µg/jour pendant 5 jours.

Après une période de 15 jours, on inocule une dose épreuve de $4,6 \cdot 10^4$ Salmonella typhimurium $C_5$ par souris. On procède de même avec un lot de 100 souris vaccinées par le vaccin vivant Stm Ra, et un lot d'animaux non vaccinés.

On obtient les résultats suivants:

3

| Fractions antigéniques | Pourcentage de survie des souris au bout de: | | Indice d'efficacité relatif: | |
|---|---|---|---|---|
| | 12 jours | 23 jours | 12 jours | 23 jours |
| PM > 50 000 | 88 | 72 | 0,9 | 0,77 |
| 10 000 < PM < 50 000 | 75 | 54 | 0,65 | 0,42 |
| PM < 10 000 | 45 | 33 | 0,09 | 0,01 |
| Vaccin vivant Stm Ra | 93 | 83 | | |
| Animaux non vaccinés | 40 | 32 | | |

L'indice d'efficacité tient compte du pourcentage de survie et de la durée moyenne de survie, le tout par rapport à un témoin.

La fraction antigénique de poids moléculaire supérieure à 50 000 daltons vaccine les souris pratiquement aussi efficacement que le vaccin vivant.

## Exemple 2

La fermentation et l'extraction s'opèrent dans les mêmes conditions que précédemment, puis on soumet le jus d'extraction à une légère centrifugation, puis à une ultrafiltration sur membranes de seuils de coupure de 50 000, 100 000 et 300 000 daltons.

On vaccine des souris par voie buccale de 200 µg par jour pendant 5 jours avec chacune des fractions obtenues. La dose d'épreuve est de $5 \cdot 10^4$ S. typhymurium $C_5$ par souris.

On obtient les résultats suivants:

| Fractions antigéniques | Pourcentage de survie des souris au bout de: | | Indice d'efficacité relatif: | |
|---|---|---|---|---|
| | 12 jours | 23 jours | 12 jours | 23 jours |
| PM > 300 000 | 30 | 30 | 0,7 | 0,7 |
| 100 000 < PM < 300 000 | 5 | 5 | 0,08 | 0,08 |
| 10 000 < PM < 100 000 | 15 | 10 | 0,3 | 0,18 |
| Smt Ra | 38 | 38 | | |
| Témoins animaux non vaccinés | 0 | 0 | | |

## Exemple 3

Les conditions de fermentation, extraction et ultrafiltration sont identiques aux conditions précédentes. La fraction antigénique de poids moléculaire supérieure à 300 000 subit une isoélectrofocalisation permettant d'obtenir différentes fractions de nature supposée être protéinique à différents points isoélectriques. Parmi toutes les fractions obtenues, l'une d'entre elles correspondant à un point isoélectrique compris entre 5,5 et 6, vaccine aussi efficacement la souris contre Salmonella typhymurium $C_5$ que le vaccin vivant.

4

0 082 072

| Fractions antigéniques | Pourcentage de survie des souris au bout de: | | Indice d'efficacité relatif: | |
|---|---|---|---|---|
| | 12 jours | 23 jours | 12 jours | 23 jours |
| PM > 300 000 | | | | |
| P. i. 5,6—6 | 50 | 20 | 0,95 | 0,45 |
| SmT Ra | 52 | 44 | | |
| Témoins animaux non vaccinés | 5 | 0 | | |

**Revendications**

1. Procédé de production par fermentation, extraction et purification d'un vaccin biochimique contre les fièvres à Salmonelles, caractérisé par la séquence préparative suivante:

— fermentation en milieu liquide adapté d'une souche avirulente à pH entre 7 et 7,7 en aérobiose moyenne, à une température comprise entre 33 et 35°C, pendant une durée de l'ordre de 24 heures;
— centrifugation, séparation du culot bactérien;
— extraction des antigènes membranaires vaccinants par mise en contact du culot bactérien avec comme solvant le tris (hydroxyméthyl) aminométhane sous agitation, à température entre 0 et 4°C, pH régulé entre 8,4 et 8,6 et pendant une durée d'au moins soixante heures;
— décantation par centrifugation, purification et fractionnement des antigènes vaccinants par ultrafiltration sur membranes de seuil de coupure choisi entre 10 000 et 300 000; dialyse de la fraction de plus faible poids moléculaire;
— et lyophilisation de la fraction antigénique vaccinante.

2. Procédé de production d'un vaccin biochimique contre les fièvres à Salmonelles, selon la revendication 1, caractérisé en ce que la souche avirulente est Salmonella thyphimurium.

3. Procédé de production d'un vaccin biochimique contre les fièvres à Salmonelles, selon la revendication 1 ou 2, caractérisé en ce que l'extraction des antigènes membranaires vaccinants est mise en oeuvre sous agitation permanente à température comprise entre 0 et +4°C, avec régulation du pH entre 8,4 et 8,6 par un acide fort, le solvant tris (hydroxyméthyl) aminométhane étant employé à concentration de 0,05 M.

4. Procédé de production d'un vaccin biochimique contre les fièvres à Salmonelles, selon la revendication 1, caractérisé en ce que l'extraction des antigènes membranaires vaccinants est conduite en présence d'un tensio-actif de la série des polyoxyéthylènes ou des polyoxyéthanols à une concentration de l'ordre de 1% par rapport au milieu extractif.

5. Procédé de production d'un vaccin biochimique contre les fièvres à Salmonelles selon une quelconque des revendications 1 à 4, caractérisé en ce que l'extrait des antigènes membranaires vaccinants après purification et fractionnement par ultrafiltration est soumis à une purification poussée par isoélectrofocalisation ou au plan industriel sur résines échangeuses d'ions.

6. Vaccin biochimique lyophilisé utilisable contre les fièvres à Salmonelles, typhoïdiques et paratyphoïdiques, obtenu selon une quelconque des revendications 1 à 5.

7. Vaccin biochimique lyophilisé utilisable contre les fièvres à Salmonelles, selon la revendication 5, caractérisé en ce que le poids moléculaire de la fraction antigénique vaccinante est supérieure à 300 000.

**Patentansprüche**

1. Verfahren zur Herstellung eines biochemischen Impfstoffes gegen Salmonellenfieber durch Gärung, Extraktion und Reinigung, gekennzeichnet durch nachstehende Verfahrensfolge:

— Gärung eines nichtvirulenten Stammes in einer geeigneten Flüssigkeit mit einem pH-Wert zwischen 7 und 7,7, in mittlerer Aerobiose, bei einer Temperatur zwischen 33 und 35°C während einer Dauer von etwa 24 Stunden;
— Zentrifugieren, Abscheidung des Bakterienrückstandes;
— Extraktion der impfenden Membranantigene durch Inkontaktbringung des Bakterienrückstandes mit dem Lösungsmittel Tri(Hydroxymethyl)-Aminomethan unter Umrühren bei einer Temperatur

5

zwischen 0 und 4°C, einem zwischen 8,4 und 8,6 eingestellten pH-Wert während einer Dauer von mindestens sechzig Stunden;
— Absetzung durch Zentrifugieren, Reinigung und Fraktionierung der impfenden Antigene durch Ultrafiltration auf Membranen mit zwischen 10 000 und 300 000 gewählter Trennschwelle; Dialyse der Fraktion mit niedrigerem Molekulargewicht;
— Lyophilisation der impfenden Antigenfraktion.

2. Verfahren zur Herstellung eines biochemischen Impfstoffes gegen Salmonellenfieber nach Anspruch 1, dadurch gekennzeichnet, daß es sich bei dem nichtvirulenten Stamm um die Salmonella thyphimurium handelt.

3. Verfahren zur Herstellung eines biochemischen Impfstoffes gegen Salmonellenfieber nach Anspruch 1 oder 2, dadurch gekennzeichnet, daß die Extraktion der impfenden Membranantigene unter ständigem Umrühren bei einer Temperatur zwischen 0 und +4°C, mit Einstellung des pH-Wertes zwischen 8,4 und 8,6 mit einer starken Säure erfolgt, wobei das Lösungsmittel Tri(Hydroxymethyl)-Aminomethan mit einer Konzentration von 0,05 M eingesetzt wird.

4. Verfahren zur Herstellung eines biochemischen Impfstoffes gegen Salmonellenfieber nach Anspruch 1, dadurch gekennzeichnet, daß die Extraktion der impfenden Membranantigene in Gegenwart eines grenzflächenaktiven Stoffes der Reihe der Polyoxyäthylene oder der Polyoxyäthanole mit einer Konzentration von etwa 1% des extrahierbaren Mediums erfolgt.

5. Verfahren zur Herstellung eines biochemischen Impfstoffes gegen Salmonellenfieber nach einem der Ansprüche 1 bis 4, dadurch gekennzeichnet, daß der impfende Membranantigenextrakt nach Reinigung und Fraktionierung durch Ultrafiltration einer eingehenden Reinigung durch Isoelektrofokussierung oder auf industrieller Ebene auf Ionenaustauscherharzen unterworfen wird.

6. Nach einem der Ansprüche 1 bis 5 erhaltener lyophilisierter biochemischer Impfstoff, der gegen Salmonellenfieber, Typhus und Paratyphus verwendet werden kann.

7. Gegen Salmonellenfieber verwendbarer lyophilisierter biochemischer Impfstoff nach Anspruch 5, dadurch gekennzeichnet, daß das Molekulargewicht der impfenden Antigenfraktion über 300 000 liegt.

## Claims

1. Process for producing a biochemical vaccine against Salmonella fevers, typhoid and paratyphoid fevers, obtained by fermentation, extraction and purification characterised by the following preparatory sequence:

— fermentation in a suitable liquid medium of an avirulent strain at a pH between 7 and 7.7, in average aerobiosis, at a temperature between 33 and 35°C, for a period about 24 hours;
— centrifuging, separation of the bacterial residue;
— extraction of vaccinating membrane antigents by putting the bacterial residue in contact with a solvent the tris (hydroxymethyl) aminoethane, with stirring, at a temperature of about 0° to 4°C, pH adjusted between 8.4 and 8.6 for a period of at least about 60 hours;
— decanting by centrifuging, purification and fractionating of vaccinating antigens by ultrafiltration on membranes cutting threshold selected between 10,000 and 300,000; dialysis of the fraction with the lower molecular weight;
— freeze-drying of the vaccinating antigen fraction.

2. Prozess for producing a biochemical vaccine against Salmonella fevers, as in claim 1, wherein the avirulent strain is Salmonella thyphimurium.

3. Process for producing a biochemical vaccine against Salmonella fevers, as claim 1 or 2, wherein extraction of the vaccinating membrane antigens is performed with constant stirring at a temperature of about 0 and 4°C, pH adjusted between 8.4 and 8.6 with a strong acid, the solvent tris (hydroxymethyl) aminomethane is used at a concentration 0.05 M.

4. Process for producing a biochemical vaccine against Salmonella fevers, as claim 1, wherein extraction of the vaccinating membrane antigens is performed in the presence of a surfactant of the class of polyoxyethylenes and polyoxyethanes in a concentration on the order about 1% in relation to the extraction medium.

5. Process for producing a biochemical vaccine against Salmonella fevers according to any one claims 1 to 4, wherein the extract of the vaccinating membrane antigens after purification and fractinality by ultrafiltration is subjected thorough purification by isoelectric focusing or on industrial level on ion exchange resins.

6. Biochemical freeze-dried vaccine used against Salmonella fevers, typhoid and paratyphoid fevers, obtained by the process of any one claims 1 to 5.

7. Biochemical freeze-dried vaccine used against Salmonella fevers, obtained by the process of claims 5, wherein the molecular weight of the vaccinating antigen fraction is greater than 300,000.